(19) **Europäisches Patentamt / European Patent Office / Office européen des brevets**

(11) **EP 2 398 394 B1**

(12) # EUROPEAN PATENT SPECIFICATION

(45) Date of publication and mention
of the grant of the patent:
**04.08.2021 Bulletin 2021/31**

(21) Application number: **10708821.3**

(22) Date of filing: **17.02.2010**

(51) Int Cl.:
**A61B 17/00** *(2006.01)*    **A61M 5/158** *(2006.01)*
**A61P 7/04** *(2006.01)*    **A61P 43/00** *(2006.01)*
**A61M 5/32** *(2006.01)*

(86) International application number:
**PCT/IL2010/000137**

(87) International publication number:
**WO 2010/095128 (26.08.2010 Gazette 2010/34)**

(54) **DEVICE FOR ADMINISTERING AN AT LEAST TWO-COMPONENT SUBSTANCE**

VORRICHTUNG ZUR VERABREICHUNG EINER AUS MINDESTENS ZWEI KOMPONENTEN BESTEHENDEN SUBSTANZ

DISPOSITIF D'ADMINISTRATION D'UNE SUBSTANCE À AU MOINS DEUX COMPOSANTS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**BA RS**

(30) Priority: **20.02.2009 US 154135 P**

(43) Date of publication of application:
**28.12.2011 Bulletin 2011/52**

(73) Proprietor: **Omrix Biopharmaceuticals Ltd.
Rehovot 76106 (IL)**

(72) Inventors:
• **ILAN, Erez
Kibbutz Netzer Sereni 70395 (IL)**

• **MERON, Moti
Herzliah 46352 (IL)**
• **REGEV, Kfir
Tel-Aviv 64293 (IL)**
• **BAR, Liliana
Rehovot 76247 (IL)**
• **NUR, Israel
Moshav Timmorim 79860 (IL)**

(74) Representative: **Kirsch, Susan Edith et al
Carpmaels & Ransford LLP
One Southampton Row
London WC1B 5HA (GB)**

(56) References cited:
**US-A- 5 211 627        US-A- 5 833 652
US-A1- 2003 225 380    US-B1- 6 733 472**

• **None**

**Description**

BACKGROUND OF THE INVENTION

Field of the invention

The present invention relates to a device for administering an at least two-component substance.

Description of the prior art

Devices for administering a substance having at least two components either by e.g. spraying or dripping onto a surface such as a surface of a body tissue of a patient or by injecting into a body tissue or organ are basically known. In particular, devices for administering an at least two-component substance are known from US-B-6,565,539, US-B-6,464,663, US-B-6,234,994, US-A-6,113,571, WO-A-2007/059801, EP-B-1 091 694, and EP-B-0 654 976.

US-A-2008/0103564, US-A-2008/0060970, US-A-2007/0,213,660 and WO-A-2008/103296 disclose a device for administering fibrin sealant into a disc. The device comprises two coaxial needles, or an outer needle and an inner catheter. The outer needle further extends the inner needle and is designed to be inserted into the patient to be treated.

WO-A-97/28834 and US-A-5,814,022 disclose a dispenser which includes two cylindrical compartments for separately containing two components. The cylindrical compartments are arranged concentrically or side by side. According to the invention the manifold can comprise separate inner and outer means wherein the inner mean extends further than the outer mean. In such an embodiment, the content of the two cylinders are conveyed into a common outlet where the two components are mixed together. The application is silent on the existence of fluid communication between the inner and outer means. EP-A-0 139 091 discloses a flash-back patency check device to indicate proper entry into a blood vessel. The catheter utilizes an over-the needle catheter with a fluid passage between the catheter and the needle and an aperture which extends through the needle. The outer catheter is not suitable for injecting a substance and the device is not intended for administering a two-component substance into a biological tissue.

By way of the known devices a substance can be administered in which one of the components may be activated by another one of the components.

The majority of the known devices comprise a multi-lumen catheter or cannula at the tip of which the components are exiting and, accordingly, are coming together outside the catheter or cannula.

US 5,833,652A discloses a component mixing catheter having a first and second lumen in coaxial arrangement.

The inner cannula is provided with holes for fluid communication between the inner and the outer lumen.

Other medical devices having multi-lumen cannulas or the like are disclosed in US-B-6,254,587, US-A-4,897,079, US-A-4,959,058, US-A-5,984,889, EP-B-0 537 573, WO-A-2008/092029, WO-A-2008/106357, and WO-A-84/04043.

Most of such devices are not adequate for mixture of the components inside the device and for ensuring minimal tissue damage and/or confined application. There is a need for a device for administering an at least two-component substance wherein the disadvantages of the prior art are overcome.

SUMMARY OF THE INVENTION

The invention relates to a device for administering a surgical sealant comprising at least two-components, wherein a first component comprises fibrinogen and a second component comprises thrombin, wherein the device comprises:

- a concentric lumen arrangement (12) including (i) an inner cannula (14) having a lumen (16) with an inlet opening at a first end and a tip with an outlet opening at a second end (62) opposite its first end, and (ii) an outer sheath (18) having opposite first and second ends facing the respective first and second ends of the inner cannula, and surrounding the inner cannula along an axial length between the first and second ends of the inner cannula,
- wherein the outer sheath at its second end (60) is sealingly and fixedly connected to the inner cannula and defines an outer lumen (28) around the inner cannula,
- wherein the second end (60) of the outer sheath is truncated and spaced apart from the tip end of the inner cannula (14), and
- wherein the inner cannula is provided with one hole (66) located 3 mm to 50 mm from the tip of the inner cannula for providing fluid communication between the outer lumen (28) around the inner cannula and the lumen of the inner cannula (16)
- wherein the device is configured such that the first and second component flow separately through the inner and outer lumen and are mixed in the inner cannula prior to being administered.

In one embodiment of the invention, the hole of the inner cannula (66) is arranged adjacent the second end of the outer sheath (60).

This design allows the use of the device according to the invention for spraying or dripping the substance onto a surface or narrow openings such as cracks and gaps or injecting the substance into a compartment with a determined volume e.g. into a biological tissue such as an intervertebral disc (IVD) which the components come into contact with each other before exiting the device.

In another embodiment, the device according to the invention further comprises at least a first (32) and a second (30) storage containers for storing the components of the substance, wherein the containers are capable of being connected to the concentric lumen arrangement (12) with the first container in fluid communication with the lumen of the inner cannula (16) and the second container in fluid communication with the outer lumen (28) around the inner cannula (14).

In still another embodiment, the device according to the invention further comprises a connecting element (20) for providing fluid communication between the containers (30, 32) and the concentric lumen arrangement (12).

In another further embodiment of the invention, the connecting element (20) comprises a first port (24) for connecting to the first container (32) and a second port (26) for connecting to the second container (30), wherein the first port is in fluid communication with the inlet opening of the inner cannula and the second port is in fluid communication with the outer lumen at the first end of the outer sheath.

In an alternative embodiment, the connecting element (20) further comprises a holding element (22) for holding the inner cannula and outer sheath of the concentric lumen arrangement, wherein the holding element comprises an inner hollow space (58) through which the concentric lumen arrangement extends, with the first end of the outer sheath located within the hollow space and the inner cannula further extending through the terminal element, and wherein a connecting channel (56) is provided starting from the second port (26) and terminating in the inner hollow space of the terminal element.

In one embodiment for dealing with more than two components of a substance to be administered, the concentric lumen arrangement further comprises at least one further sheath (68) surrounding the outer sheath (18) along an axial length thereof and comprising first and second ends facing the first and second ends of the outer sheath, respectively, wherein the further sheath is sealingly connected (61) to the outer sheath (18) adjacent the second end thereof and defines a further lumen (70) around the outer sheath, and wherein the outer sheath is provided with at least a second opening (72) providing fluid communication between the further outer lumen (70) around the outer sheath (18) and the lumen (28) within the outer sheath around the inner cannula, and wherein at least a one further storage container is provided for storing a further component of the substance and capable of being connected to the first end of the at least one further sheath of the concentric lumen arrangement.

Yet in another embodiment of the invention, the device further comprises a pressing mechanism (46) for pressing the components of the substance out of the containers (30, 32) to the concentric lumen arrangement (12) and further there through out of the tip at the second end of the inner cannula (62).

Yet in another further embodiment of the invention, the at least one component is activated by the at least second component of the substance.

In another embodiment of the invention, the at least one component comprises fibrinogen, and the at least second component comprises thrombin.

Still in another embodiment of the invention, the administering is carried out by injection.

Still in another further embodiment of the invention, the administering is carried out by spraying or dripping.

Disclosed herein is a method of administering to a subject in need a substance comprising at least two components, the method comprising the steps of:

- providing a device according to the invention,
- piercing the tip of the inner cannula of the concentric lumen arrangement into a biological tissue, and
- administering the at least two component substance in a mixed condition through the tip of the inner cannula of the concentric lumen arrangement.

In one embodiment of the invention, the substance is a fibrin sealant with a fibrinogen component and a thrombin component, wherein examples of a thrombin component which can be used in the fibrin sealant is described in EP-B-0 534 178, WO-A-93/ 05822, and EP-B-0 378 798.

One of the components of the substance can be a biologically active component (BAC) as described in US-B-6,121,232 and WO-A-98/33533, wherein the plasmin(ogen) was removed as described in EP-B-1 390 485 and tranexamic acid was not added (US-B-7,641,918).

In another alternative, the substance is a drug with a drug precursor component and a drug precursor activator component.

Also disclosed is a method of administering to a

subject in need a substance comprising at least two components, the method comprising the steps of:

- providing a device according to the invention, and
- administering onto a biological tissue the at least two component substance in a mixed condition through the tip of the inner cannula of the concentric lumen arrangement, wherein the step of administering is carried out by dripping or spraying.

An exemplary method of administering of the substance is by way of spraying. In such a method, one of the components can be a gas, e.g. air.

Also disclosed is a method of treating a tissue defect in a subject, comprising the steps of:

- providing a device according to the invention, and
- administering a substance comprising at least two components in a mixed condition to the patient.

The defect may be selected from the group consisting of renal defects; fistulas; ulcers; bleeding; fissures or cracks in the dura; cerebrospinal fluid leakage; spine disease, disorder or condition such as spinal disc defects, full tears and fissures in the annulus fibrosus, punctures in the annulus fibrosus, fissures or cracks in the intervertebral disc or a reduction in the height of the intervertebral discs; cardiovascular disease; heart disease; burn eschars; seromas such as seromas following mastectomy; hemorrhoids; air leaks such as in pulmonary resection; malignant pleural effusion; cystitis; and anastomosis such as ureteral anastomosis or intestinal anastomosis.

In another embodiment of the invention, the substance is a sealant.

In another further embodiment of the invention, the sealant is a fibrin sealant. The invention relates to the use of a device according to the invention for administering onto a surface a substance comprising at least two components in a mixed condition through the tip of the inner cannula of the concentric lumen arrangement.

In one embodiment of the invention, the administering is carried out by dripping or spraying.

In another embodiment of the invention, the surface is a surface of a body part of a patient.

Yet in another embodiment of the invention, the substance is a fibrin sealant with a fibrinogen component and a thrombin component.

In a further embodiment of the invention, the substance is a drug with a drug precursor component and a drug precursor activator component.

BRIEF DESCRIPTION OF THE DRAWINGS

The invention will be described in more detail referring to the drawing in which

Fig. 1 shows an embodiment of the device according to the invention having a concentric double-lumen cannula with storage containers for two components of a substance to be administered attached and further providing a manually operable pressing mechanism.

Fig. 2 shows an embodiment of the device.

Fig. 3 shows a further enlarged view of area III of Fig. 2.

Fig. 4 shows an embodiment of the concentric lumen arrangement comprising three concentric lumens for administering a three component substance.

Fig. 5 shows an embodiment of the concentric lumen arrangement comprising four concentric lumens for administering a four component substance.

Fig. 6 shows an embodiment of the concentric lumen arrangement comprising a solid stylet.

Fig. 7 shows the height recovery of isolated and emptied-discs following fibrin sealant injection into the IVD space. The measurements were carried out in different puncture diameters (1.6 through 3.5 mm). The results are presented as percentage of the maximal compression possible of an untreated disc in the same experiment (100 %).

Fig. 8 shows the localization of fibrin sealant following injection into isolated intervertebral discs having full annular tears (A) and annular fissures simulating grade 1 and 2 degenerative discs (B). The injection procedure was carried out using a needle having an outer diameter of 0.8192 mm (21 G needle). The localization of the sealant is marked with an arrow.

Fig. 9 shows immunostaining of fibrin in a representative section obtained from an IVD injected with the fibrin sealant components in an *in-vivo* setting (A). A stained casted fibrin clot prepared *in vitro* was used as the control (B). Fig. 9C shows immunostaining of fibrin in a representative section obtained from an IVD injected with the fibrin sealant components in a second study preformed. Fibrin staining is marked with an arrow.

Fig. 10 shows the percent of punctures which led to annulotomy (annulus fibrosus tissue removing; A) and the excised tissue weight per stabbing (B) when puncturing the annulus fibrosus tissue with needles having the following outer diameters: 0.8192, 1.067, 1.270, and 1.651 mm (21, 19, 18, and 16G, respectively). (C) Representative pictures of the intervertebral disc sections following the puncturing procedure. The ruptures in the annulus tissue are marked with an arrow.

Fig. 11 shows the flow rates achieved in concentric needles having the following outer diameter dimensions: 0.6414mm/1.270mm (23G/18G) (inner/outer) and 0.8192mm/1.651mm (21G/16G) at the different forces. N=Newton

Fig. 12 shows the maximal force needed to overcome needle clogging in concentric needles having the following outer diameter dimensions: 0.6414mm/1.270mm (23G/18G; A) and 0.8192mm/1.651mm (21G/16G; B). The maximal force was recorded when the injection was resumed following the clogging.

Fig. 13 shows an alternative embodiment of the device wherein the second end of the outer sheath is sealingly and fixedly connected to the inner cannula by means of a supplemental part.

Fig. 14 shows a further enlarged cross-sectional view of area I of Fig. 13.

Fig. 15 shows various embodiments of a cross sectional view of the concentric lumen arrangement at the marked point II in Fig. 14. A shows an outer lumen surrounding an inner lumen according to the invention. B shows a concentric lumen arrangement having an outer cannula with two counter lumens.

## DESCRIPTION OF PREFERRED EMBODIMENTS

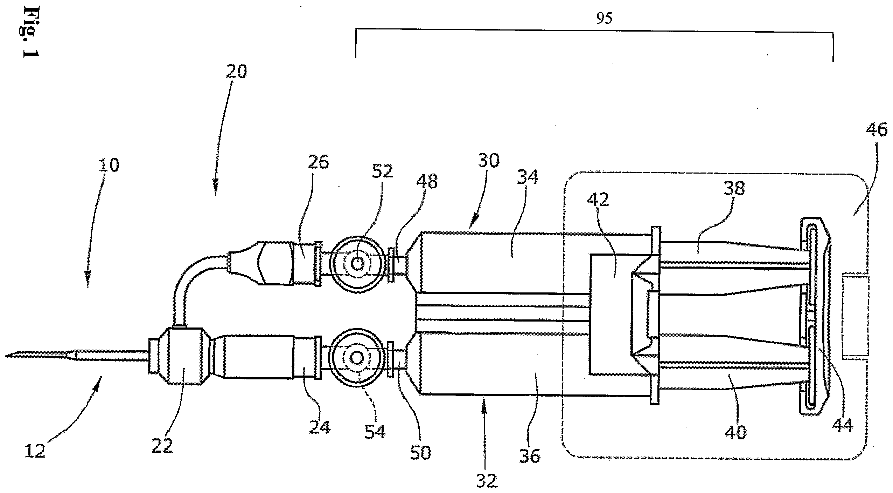

Diverse views of a first embodiment of the device for administering a two-component substance according to the invention are depicted in Figs. 1 to 3. The device 10 comprises a concentric double-lumen arrangement 12 comprising an inner cannula 14 defining an inner lumen 16 and an outer sheath or cannula 18 both attached to a connecting element 20 comprising a holding element 22. The connecting element 20 is provided with two ports 24, 26 with the first port 24 being in fluid connection with the first or inner lumen 16 of the inner cannula 14 and the second port 26 being in fluid connection with an outer lumen 28 formed as an annular space around the inner cannula 14 and radially limited by the outer sheath or cannula 18.

The two ports 24, 26 are in fluid connection with the storage containers 30, 32 for e.g. storing the components to be administered (e.g. sprayed or dripped onto or injected into a biological tissue or the like) by means of the device 10. In this embodiment the storage containers 30, 32 are formed as syringes having a hollow cylinder 34, 36 and associated plungers 38, 40. In order to manually operate both syringes simultaneously, the cylinders 34, 36 are connected by a first connecting element 42 and the plungers 38, 40 are connected by a second connecting element 44 as basically known in the art (see e.g. US-B-6,234,994). Also, an automatically or manually operable pressing mechanism 46 for pressing the plungers 38, 40 into the cylinders 34, 36 can be used. These pressing mechanisms are generally known in the art (see e.g. US-A-2008/0103564, US-A-2008/0060970, US-B-6,464,663, and US-B-6,565,539). The outlets of the cylinders 34, 36 can be directly attached to the first and second ports 24, 26 of the connecting element 20 or can be indirectly attached e.g. by employing fluid control devices 52, 54 arranged between the outlets 48, 50 and the ports 24, 26. These fluid control devices 52, 54 can be used for sucking the components out of vessels (not shown) attached to fluid control devices and into the syringes in a first step and for discharging the components out of the syringes through the connecting element 20 and the concentric lumen arrangement 12 for administering purpose. Examples for the fluid control devices which can be used for the device according to the invention are described in EP-B-0 814 866 and US-A-6,113,571.

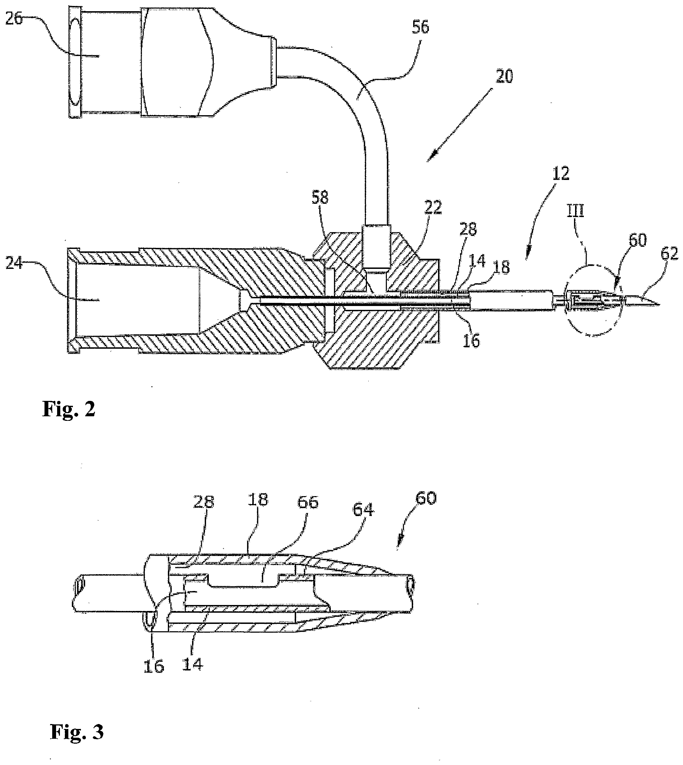

As shown in further details in Figs. 2 and 3, the second port 26 of the connecting element 20 is in fluid communication with the holding element 22 by means of a connecting channel e.g. a bent tube 56 leading to an inner hollow space 58 through which the double-lumen arrangement 12 extends and is fixed. The hollow space 58 is in fluid communication with the outer annular lumen 28.

The outer cannula or sheath 18 is truncated at its free end 60 which is spaced apart from the tip end 62 of the inner cannula 14. At its truncated end 60, the outer sheath or cannula 18 is fixedly attached to the outer surface of the inner cannula 14. This fixed connection of the two cannulas forms a sealed connection. Within the area of the inner cannula extending through the outer sheath or cannula 18, the wall 64 of the inner cannula 14 is provided with at least one hole or opening 66 through which the inner lumen 16 and the outer lumen 28 are in fluid communication. Accordingly, the component flowing via the second port 26 and further through the tube 56 as well as through the hollow space 58 and further through the outer lumen 28 comes into contact with the other component flowing through the inner lumen 16 of the inner cannula 14 so that downstream of the at least one hole 66 both components are flowing through the inner cannula 14 to be mixed with each other and so as to be discharged out of the tip end 62 as a mixture.

The term "mixed" refers to the coming together of the components. The mixing can result in a completely, substantially, or partially homogenous mixture.

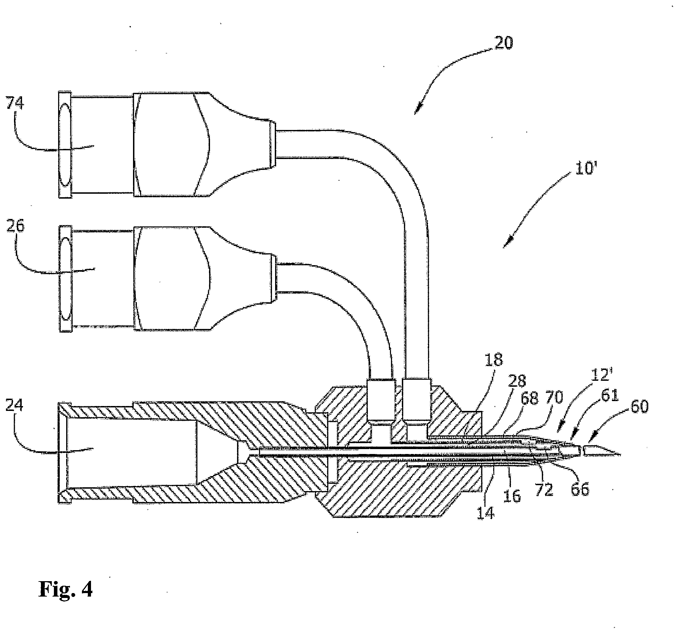

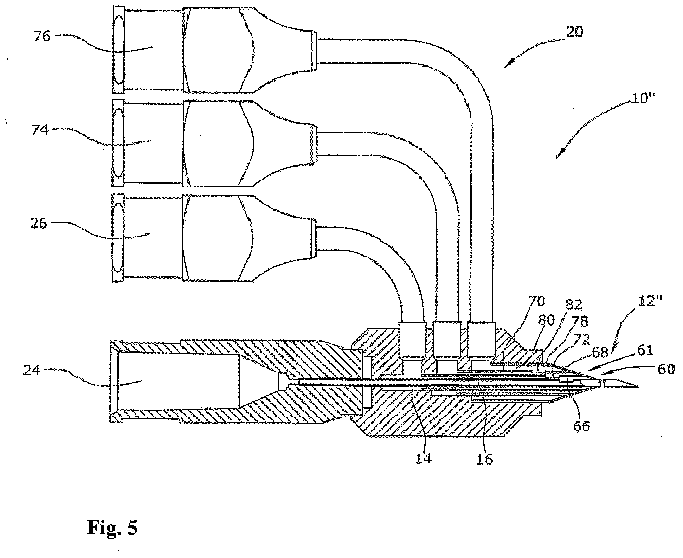

Alternative embodiments of devices 10' for administering a three-component substance and a device 10" for administering a four-component substance are shown in Figs. 4 and 5. The device 10' comprises a three-lumen arrangement 12' having inner cannula 14 defining an inner lumen 16, an intermediate sheath or cannula 18 defining an intermediate annular lumen 28, and an outer sheath or cannula 68 defining an outer annular lumen 70. The cannulas are sealingly connected at there free ends 60, 61 wherein at least first opening 66 in the inner cannula 14 provides fluid communication between the inner lumen 16 and the intermediate or middle lumen 28 while at least a second opening 72 provides fluid communication between the middle lumen 28 and the outer lumen 70. The connecting element 20 of the device 10' comprises three ports 24, 26, 74 which are in fluid communication with the afore-mentioned lumens of the three-lumen arrangement 12' as shown in Fig. 4.

In Fig. 5, the device 10" comprises a connecting element 20 having four ports 24, 26, 74, 76 through which four components of a substance to be administered are applied. The device 10" further comprises a four-lumen arrangement 12" which is designed similar to the three-lumen and double-lumen arrangements 12' and 12 of Figs. 2 to 4 and includes a further outer cannula or sheath 78 defining a further outer annular lumen 80 in fluid communication with the next inner annular lumen 70 through at least one hole 82 in the next inner cannula or sheath 68.

A device was designed to enable application of an at least two-component substance where one of the components may be activated by another component. The substance can be applied to a fixed location e.g. an external surface of the body or a location within the body. The location within the body can be a closed compartment which is filled with a biological material such as cells and/or extra cellular matrix; liquid and/or gas. In such an embodiment the administration can be carried out by injection.

Alternatively, the location within the body can be a surface of an internal body part. In such an embodiment the administration can be carried out by spraying or dripping.

The device comprises at least two concentric needles or cannulas that enable separate feeding for each of the at least two components (see Figs. 4 and 5). In one embodiment of the invention, the device comprises two concentric needles or cannulas. The outer cannula or sheath terminates at a short distance from the tip of the inner cannula wherein at this location the outer cannula is sealingly and fixedly connected (e.g. by welding or gluing) to the inner cannula.

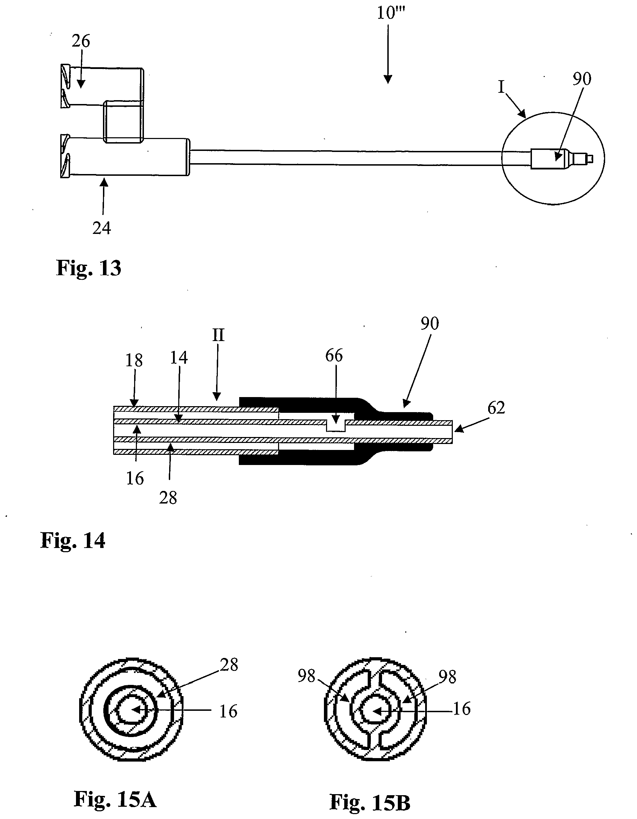

Alternative embodiments of devices 10'" is shown in Fig. 13 wherein the second end of the outer sheath is sealingly and fixedly connected to the inner cannula by means of a supplemental part 90. Fig. 14 shows a further enlarged cross-sectional view of area I of Fig. 13. Advantageously, such a device has a small diameter flexible concentric arrangement which enables administration of the substance onto a confined surface by dripping. The use of such a device is of advantage for administering a homogeneous mixture of the substance to remote and narrow areas within the body, especially when performing a laparoscopic procedure, for example, in closure of the dura to reduce and/or minimize cerebrospinal fluid (CSF) leaks.

The concentric lumen arrangement can be formed by extrusion and have a multi-lumen outer cannula. Fig. 15 shows various embodiments of a cross sectional view of the concentric lumen arrangement at the marked point II in Fig. 14. A shows an outer lumen surrounding an inner lumen according to the invention. B shows a concentric lumen arrangement having an outer cannula with two counter lumens 98.

The inner cannula comprises, at a short distance from the sealing point of the cannulas, at least one hole 66. This or each hole in the inner cannula provides a fluid communication between the outer lumen and the inner lumen. The components flow separately through the inner and outer lumen and are mixed in the inner cannula immediately prior to being applied into the selected target e.g. tissue or region. The complete mixing is carried out within the inner cannula downstream of the hole.

In one embodiment of the invention, the fluid flow rate is from about 0.5 to about 10 ml/min such as 0.5, 1, 2.5, 5, 10 ml/min.

The inner diameter of the inner cannula may range from about 0.0035 to about 2 mm. In one embodiment of the invention, the inner diameter of the inner cannula is about 0.495 mm. Advantageously, adequate flow in the concentric needles is obtained when the distance between the outer diameter of the inner needle and the inner diameter of the outer needle is more than about 0.05 mm e.g. 0.1, 0.15, 0.2, 0.25, 0.3, 0.4, 0.5 mm and so on. In one embodiment of the invention, the distance between the outer diameter of the inner needle and the inner diameter of the outer needle is about 0.2 mm. In another embodiment of the invention, the distance between the outer diameter of the inner needle and the inner diameter of the outer needle is about 0.49 mm.

In another embodiment of the invention, the device comprises three concentric needles for the administration e.g. by injection of a third component such as a contrast agent, a gas, or a cell composition. The contrast agent may be chosen from various non-toxic agents, such as iodine based contrast agent, for example Iopamiro™.

The device can be used to apply substances which are not stable when mixed or substances which need to be held separately until application.

It was found according to the invention that in intradiscal administering of a two component fibrin sealant, fibrinogen and thrombin, the needle which penetrates through the annulus fibrosus (AF) and into the nucleus pulposus (NP) needs to be as thin as possible in order to inflict as little damage as possible. Damage inflicted to the disc during the injection procedure can lead to fissures and ruptures of the AF and leakage of NP material from the disc. It was found according to the invention that the device design permits minimal damage to a target tissue, at any desired location.

The sealing and the fixed connection of the two cannulas can be carried out using various techniques such as tight fit (press fit) clamping, gluing, laser welding, cold forming, hot forming or by any other method known in the art which forms sealing.

The device of the invention can be used to administer a substance made of at least two components into or onto an injured tissue or region in any medical application such as, but not limited to, urology, for example, for nephrectomy, traumatic renal reconstruction, and fistula repair; diabetic ulcers; chronic ulcers; ulcers, for example, as hemostat and to accelerate healing; occlusion of varicosis; occlusion of varicose veins; filling the fistulas space; internal disc disruption; for treating spine disease, disorder or condition; neurosurgery such as for sealing fissures or cracks in the dura (closure of the dura) and reducing or preventing CSF leakage; sealing full tears and fissures in the annulus fibrosus; sealing punctures in the annulus fibrosus; for filling and sealing of the IVD; for IVD height recovery or restoration; cardiac operations; to debride burn eschars; mastectomy such as for treating seromas; clogging hemorrhoids; pulmonary resection such as for prevention and treatment of air leaks; remission of malignant pleural effusion; for filling up cystitis ;and for ureteral anastomosis. Another example is the administration of a drug precursor as a first component and an activator as the second component into a malignant tissue.

The device may be used for treating spine disease, disorder or condition.

The term "spine disease, disorder or condition" refers e.g. to intervertebral disc and/or to central nervous system disease, disorder or condition.

The term "intervertebral disc disease, disorder or condition" refers to multiple disorder, disease or conditions involving intervertebral disc degeneration and/or injury such as disc herniation, fissured disc, spinal stenosis, black disc, disc pain, etc.

Oftentimes, the term "intervertebral disc disease, disorder or condition" is used as synonymous with the term "Degenerative Disc Disease (DDD)" or "Internal Disc Disruption (IDD)".

The fibrin sealant may be applied into the NP area to restore or increase the height of the disc. The applied fibrin sealant is able to resist compressive loads similarly to the natural NP tissue. The height of the disc may decrease as a result of NP material leakage through full tears or tiny fissures present in the AF. The fibrin sealant can be used to fill any cracks, voids or openings in the AF.

The reduction in the height of the disc can occur regardless of the presence or absence of tears or fissures in the AF. The height may be reduced due to nucleus pulposus dehydration and degradation of the collagenous fibers.

In one embodiment of the invention, the device is employed for administering fibrin sealant for filling voids in the AF following intradiscal electrothermal treatment (Derby and Kim BJ. "Effect of intradiscal electrothermal treatment with a short heating catheter and fibrin on discogenic low back pain". Am J Phys Med Rehabil. 2005;84:560-561).

The device may be used to administer fibrin sealant for filling voids in the NP. For instance the device may be used for administration of fibrin sealant to simulate the structure, physical properties and biomechanical function of the NP.

The fibrin sealant can be applied into the damaged disc, without removing the NP. Alternatively, prior to administering the fibrin sealant all or a portion of the NP is excised. The removal procedure can be carried out enzymatically by disrupting the extra cellular matrix, mechanically, by using a Nucleotome probe and/or by any other method known in the art.

The proteolytic enzymes capable of degrading cartilage tissue include, but are not limited to, serine peptidases, for example, trypsin, chymotrypsin, pancreatic elastase; cystein peptidases, for example, papain chymopapain; aspartic peptidases, for example, pepsin, metallo peptidases, for example, collagenase, gelatinase, pronase, chondroitinase; hyaluronidase and/or alternative chemical materials which would degrade disc material in the same or similar manner, and combinations thereof.

As used herein the term "central nervous system disease, disorder or condition" refers to any disease, disorder, or trauma that disrupts the normal function or communication of the brain or spinal cord. Without limitation, there may be mentioned brain and spinal cord injuries due to neurosurgery, trauma, ischemia, hypoxia, neurodegenerative disease, metabolic disorder, infectious disease, compression of the intervertebral disc, tumors and autoimmune disease and CSF leakage.

The substance can be a surgical sealant. Different types of surgical sealants can be used, including, but not limited to, a biological sealant (made from clotting proteins e.g. fibrinogen and thrombin and/or other natural products); a synthetic sealant such as acrylates, cyanoacrylates, and polyethylene glycol (PEG) polymers; and a semisynthetic sealant e.g. made from a combination of biological and synthetic materials such as gelatin-formaldehyde-resorcinol (GFR) glue. In one embodiment of the invention, the substance is a synthetic sealant such as described in WO-A-2006/086510. In another embodiment of the invention, the substance is a fibrin sealant.

Exemplary of a two component-substance include, but are not limited to, fibrinogen and thrombin, alginate and calcium, chondroitin sulphate and an acid such as hyaluronic acid, antigen and adjuvant, two components which form a colloidal suspension, two components which enables the formation of liposomes, two components in which one requires activation by the other, two components in which one component will activate the other component, mixing of any two liquids. In one embodiment of the invention, the two components of the substance are fibrinogen and thrombin. In such an embodiment, when the two components are mixed polymerization process is activated and application of the sealant is advantageously carried out without delay before resistance to flow becomes excessive.

In one embodiment of the invention, one component is more viscous than the other. In another embodiment of the invention, the viscous component is transferred in the inner lumen. To facilitate feeding of the viscous component throughout the inner lumen, the inner diameter of the inner cannula can be increased e.g. at the expense of the inner diameter of the outer cannula. In a further embodiment of the invention, the two component substances are BAC and thrombin. Yet, in another embodiment of the invention, the BAC component is fed through the inner lumen and the thrombin component through the outer lumen. Alternatively, the BAC component can be fed through the outer lumen and the thrombin component through the inner lumen.

Varying the amount of thrombin reduces or lengthens the time required for polymerization. Typically, increased concentrations of thrombin per unit amount of fibrinogen results in faster fibrin formation which can result in premature clot formation and clogging of the needle. The unique design of the device minimizes this problem since the two components flow together in a relatively short distance as compared to other spinal or epidural needles; it was shown in one setting, that when a partially set up clot was allowed to be formed within the inner needle (by delay of about two minutes in the injection procedure) the clot was successfully expelled out of the needle allowing delivery of additional substance. The force required to unclog the concentric needle of the invention is significantly lower as compared to other needles of the same gauge in which the joint flow is longer. In one embodiment of the invention, the concentration of thrombin used is about 1000 IU/ml. In another embodiment of the invention, the concentration of thrombin used is about 100 IU/ml.

A device comprising a concentric lumen arrangement according to the invention enables application of at least a two component-substance. In one embodiment of the invention, a two component substance where the first component may be activated by the second component is applied. The two component-substance can be applied to a fixed location e.g. within the body. The concentric lumen arrangement according to the invention can be used with any applicator-device designed for application of two or more components such as the one described in WO-A-2007/059801 excluding the Y mixing module.

In one embodiment of the invention, the inner needle of the concentric lumen arrangement has an outer diameter of 0.8192 mm (21G) and the outer needle has an outer diameter of 1.651 mm (16G). In another embodiment of the invention, the inner needle has an outer diameter of 0.6414 mm (23G) and the outer needle has an outer diameter of 1.270 mm (18G). In another further embodiment of the invention, the inner needle has an outer diameter of 0.6414 mm (23G) and the outer needle has an outer diameter of 1.651 mm (16G).

The length of the concentric needle/lumen arrangement 12 can be from about 10 to about 700 mm. In one embodiment of the invention, the length is from about 20 to about 500 mm. In another embodiment of the invention, the length is 300 mm. In another further embodiment of the invention, the length is 180 mm. Yet, in another further embodiment of the invention the length is 90 mm. The outer needle terminates at a site spaced from the tip of the inner needle. The projecting inner needle can penetrate into the desired location. In one embodiment of the invention, the length of the projection of the inner needle allows penetration into a tissue within the body. In another embodiment of the invention, the device is used for intradiscal injection and the inner needle terminates 12 mm after the outer needle. In another further embodiment of the invention, the inner cannula terminates 3 to 50 mm after the outer cannula.

The distance between the center of port 24 and the center of port 26 can be suited to the distance between the outlets 48, 50 of the applicator device 95. In one embodiment of the invention, an applicator device as described in WO-A-2007/059801 is used and the distance between the centers of the two ports 24, 26 is 18 mm.

The needles/cannulas may be formed from any material e.g. a biocompatible material. In one embodiment of the invention, the material used is rigid and enables penetration into tissues such as soft tissues or bones. The needles can be resistant to deformation which can occur during penetration of the substance into the tissue. Alternatively, the needles/cannulas can be made of flexible materials. Several materials can be used, including, but not limited to, metal including stainless steel of various types, alloys, polymers such as polyvinylchloride (PVC), polytetrafluoroethylene (PTFE), polyethersulfone (PES), polyethylene (PE; of various types), polyurethane (PU), Ultem® (polyetherimide; PEI), polycarbonate (PC), polyetheretherketone (PEEK), polysulfone (PS), polypropylene (PP) and a combination thereof.

The outer surface of the projecting inner needle can be rotated. In the inner needle there is provided one or more holes. Complete mixing of the components is carried out within the inner needle downstream to the hole(s). For optimal fibrin sealant delivery, the distance between the hole(s) to the tip of the projecting inner needle allows formation of a homogenous mixture and at the same time allows the substance to be expelled from the needle before clogging occurs and resistance to flow becomes excessive. Also, for optimal results the thrombin concentration and flow rate can be varied. In the invention, there is provided one hole which is located 3 to 50 mm such as 20 mm from the end of the tip of the projecting inner needle. The device may be used for intradiscal injection and is resistant to a pressure of up to about 689,474 Pa (100 PSI) such as 344,737 Pa (50 PSI).

The device can contain additional cannula(s) or needle(s) for the injection of a contrasting agent and/or a cell composition such as shown in Fig. 4 and 5.

A device comprising a concentric needle arrangement according to the invention can also comprise one or more of the following parts:

1. Two connective tubes with luer lock at ends (female and male) can be attached between the outlets 48, 50 and the ports 24, 26 or between the fluid control devices 52, 54 and the ports 24, 26. The connective tubing enables the operator to freely move the applicator device without causing movement of the needle within the tissue, thereby minimizing the risk of possible tissue damage and/or maximizing the device's ease of use.

2. An automatically or manually pressing mechanism which enables to apply a gradual and continuous force to the applicator device see e.g. 46 in Fig. 1. In one embodiment of the invention, the pressing mechanism utilizes a screw.

3. A force or pressure measuring unit which enables to monitor the pressure inflicted to the injured site.

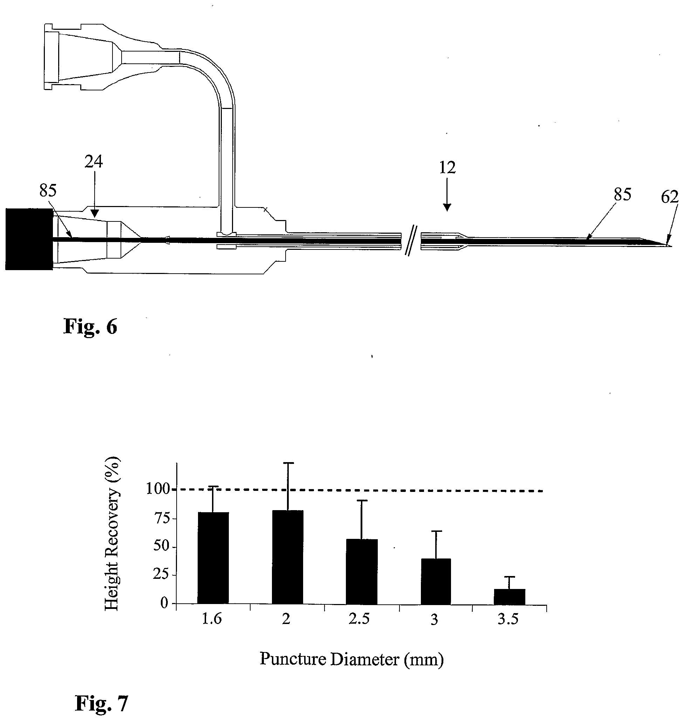

4. A solid stylet can be added into the concentric lumen arrangement (e.g. Fig 6). Having such a stylet may prevent, or substantially prevent, the accumulation of non-target tissue such as skin and subcutaneous tissue within the lumen of the inner needle as the assembly is advanced in the body into the location of interest. Consequently, the risk of infection due to penetration of non-target tissue into the site of interest during the administration procedure is reduced. Advantageously, the stylet may also reinforce and support the concentric needle arrangement in the terminal element and prevent bending or breaking of the concentric needles. The stylet can be positioned in the lumen of the inner needle and may extend all the way through the inner needle arrangement. The stylet can further extend through the terminal element, up to the first end of the inner needle or up to the end of the first port 24. In Fig. 6, the concentric needle arrangement 12 comprises a solid stylet 85 which is positioned within the lumen of the inner needle. The stylet can be made from any substance including, but not limited to, metal including stainless steel of various types, alloys, polymers such as polyvinylchloride (PVC), polytetrafluoroethylene (PTFE), polyethersulfone (PES), polyethylene (PE; of various types), polyurethane (PU), Ultem® (polyetherimide; PEI), polycarbonate (PC), polyetheretherketone (PEEK), polysulfone (PS), polypropylene (PP) and a combination thereof.

The term "terminal element" refers to the T-connection between the first end of the outer sheath 18 which is located within the inner hollow space 58 and the inner lumen 16.

The connecting element 20 and the applicator-device 95 may be attached by adapters, such as connective tubes with male and female luer lock at ends or other such adapters known in the art.

The length of the connective tubing can be up to about 60 cm. The inner diameter of the connective tube can be from about 0.5 to about 5 mm. The connective tubing can be formed from any material which enables flexible connection between the applicator device and the connecting element 20, including, but not limited to, silicon, polyvinylchloride (PVC), polypropylene (PP), polyethylene (PE; of various types), polytetrafluoroethylene (PTFE) and a combination thereof.

In one embodiment of the invention, the connective tubing withstands a pressure of at least 344,737 Pa (50 PSI).

The pressing mechanism device, if present, enables to apply a gradual and continuous force to the applicator device. In one embodiment of the invention, the pressing mechanism enables to apply a continuous force against the back pressure of the intervertebral disc without releasing the build pressure.

The pressing mechanism can be provided in different shapes or sizes. The pressing mechanism can contain all or a part of the applicator device. In the former, the pressing mechanism can enable visualization of the graduated syringes.

The pressing mechanism can be made of metal such as stainless steel, alloy, polymers such as polyvinylchloride (PVC), polyethersulfone (PES), polyethylene (PE; of various types), polyurethane (PU), Ultem® (polyetherimide; PEI), polycarbonate (PC), polyetheretherketone (PEEK), polysulfone (PS), polypropylene (PP), acrylonitrile butadiene styrene (ABS) plastic, and a combination thereof.

The pressure applied during the administration of the substance can be monitored indirectly by a force measuring unit such as a load cell and/or a strain gauge. The force measuring unit can be in any size or shape. In one embodiment of the invention, the applied pressure is monitored indirectly by a cylinder load cell having a diameter of 10 mm and a height of 7 mm.

The pressure can also be monitored directly by a pressure sensor measuring unit such as an analog or digital pressure gauge. The pressure sensor measuring unit can be placed on the fluid flow line, for example, by extension tubing, or to any other part of the fluid conducting pathway. Direct measurement can also be carried out by using an appropriate pressure transducer element which converts force information into pressure measurement data. The pressure transducer element can be placed on the fluid flow line.

The force measuring device and the pressure sensor unit can comprise a visual display monitor unit such as, but not limited to, 7 segment, LCD, computer screen and any other visualization unit known in the art.

The force or pressure measuring unit can be housed within the pressing mechanism.

The components of the device may be for a single patient use or can be sterilized for repeated use with multiple patients. The components can be pre-sterilized by different methods known in the art including, but not limited to, gamma radiation, steam sterilization, chemical vapor, high-voltage electron beam radiation and the like. In one embodiment of the invention, the device components are sterilized using steam sterilization.

The following examples are illustrative but not limiting.

EXAMPLES

**Example 1: Concentric Needle**

Concentric needles were assembled using spinal needles having the following outer diameter dimensions 1.270 mm (18G), 0.8192 mm (21G), 0.6414 mm (23G) ("phoenix" spinal needles sterilized by ethylene oxide Kobayashi Shoji K.K Tokyo, Japan); and 1.651 mm (16G) (Sigma cat. No. Z100897-1EA). The length of the needles was 90 mm.

Two concentric needles having the following outer diameter dimensions were assembled: 0.8192mm/1.651mm (21G/16G) and 0.6414mm/1.270mm (23G/18G) (inner/outer). A delarin plastic bushing was used to connect the inner and the outer needles. The two needles were sealed using polyethylene glue. The mixing hole was located in the inner needle in a distance of 20 mm from the distal tip end of the projecting inner needle i.e. the two components flow together in a distance of 20 mm until they were expelled from the needle. The assembled concentric lumen arrangement as used in these experiments is shown in Figs. 2 and 3.

**Example 2: Two Component Fibrin Sealant Applied with the Concentric Needle**

1000 IU/ml thrombin of a two component fibrin sealant (like the one described in EP-B-0 378 798) and BAC (prepared as described in US-B-6,121,232 and WO-A-98/33533, wherein the plasmin(ogen) was removed as described in EP-B-1,390,485 and tranexamic acid was not added) were used. The thrombin component was diluted 10-fold with a dilution buffer [0.4 M CaCl$_2$ in DDW (Riedel-de Haen cat No 31307) diluted 1:10 in saline to a final concentration of 0.04 M] directly before use. The BAC component was used as is. In all the experiments described below, the thrombin component was administered through the outer needle and the BAC component was administered through the inner needle.

**Example 3: The Effect of the Puncture Diameter on the Height Recovery of the Disc Following Compressive Load**

The efficacy of the concentric needle was monitored in intradiscal administration of a two component fibrin sealant, fibrinogen and thrombin. One of the purposes of injecting fibrin sealant into the intervertebral disc is to restore or increase the height of the disc. In such administration, the needle is injected through the annulus fibrosus (AF) and into the nucleus pulposus (NP) area. The puncture diameter inflicted during the injection procedure can cause significant disc injury such as fissures and ruptures of the AF and subsequent NP material leakage from the disc. The disc is subjected to load thus, there is a need to evaluate which puncture diameter does not inflict significant damage to the disc and enables height recovery of the disc following compressive load. Accordingly, the following experiment was carried out to monitor the effect of puncture diameter on the ability of the disc to retain its original height following compressive load. Pig isolated lumbar spine (L1-L5; Lahav, Israel) was cleaned from muscle tissue and the lumbar vertebral bodies were cut in the middle using an electric bone saw to obtain isolated intervertebral discs. Afterwards, the isolated intervertebral discs were flattened using an electric sander to produce smooth, parallel symmetrical upper and lower surfaces. The isolated intervertebral discs were placed in a tension and compression testing machine (LF plus, LLOYD instruments Ltd, Hampshire, UK) and compression was measured at 500 N. Then, the NP tissue was enzymatically digested by injection of a solution containing 6 mg/ml collagenase and 2 mg/ml hyaluronidase in PBS (0.5 ml; Sigma cat No C-6885 and H-2126, respectively). After 30 minutes, two syringes connected to needles having an outer diameter of 1.651 mm (16G) were inserted at opposite ends of the isolated IVDs and about 2 ml PBS was injected into the IVD. The disc space was emptied and filled several times by transferring the disc content between the two opposite syringes and then the IVD content was discarded. PBS injection and emptying of the IVD content as described was carried out three times. This procedure was repeated once again using about 2 ml EDTA (10 mM; Riedel-de-Haen cat No 34549).

Then, each emptied-disc was punctured 4 times using a needle with an outer diameter of 1.651 mm (a 16G needle) or different drills resulting in holes of different diameters (1.6, 2, 2.5, and 3.5 mm). The compression of each emptied-disc was then measured at 500 N.

Afterwards, the punctured-emptied discs were injected with fibrin sealant (as described above; applied simultaneously at equivalent volumes) using Omrix's injection device as described in WO-A-2007/059801 excluding the Y mixing module. The components were injected through the existing punctures until excess solution was spilt out of the injection site. The re-filled intervertebral discs were incubated at room temperature for about 30 min until a clot was formed. Following the incubation period, compression measurements of the filled disc were carried out at 500 N.

All measurements were repeated 5 times. For each individual IVD the height recovery was calculated as the percentage from the maximal possible compression value (considered as 100%) in order to exclude disc variability and ensure standardization. The maximal value is calculated by subtracting the compression value of emptied-disc under 500 N load from the compression value of untreated (naive) disc under 500 N load (the denominator in the below formula). The numerator expresses the compression value of the re-filled disc.

The height recovery ability of the fibrin-filled disc was calculated according to the following formula:

$$\frac{\text{(Compression of untreated disc under 500 N load)} - \text{(Compression of re-filled disc under 500 N load)}}{\text{(Compression of untreated disc under 500 N load)} - \text{(Compression of emptied-disc under 500 N load)}} \times 100$$

Fig. 7 shows the height recovery ability of emptied-discs following fibrin sealant injection into the nuclear space. The measurements were carried out in different puncture diameters (1.6 through 3.5 mm). The results are presented as percentage of the maximal possible compression value (100%) as explained above.

The results show that 3 and 3.5 mm puncture diameters resulted in a significant loss of disc height following compressive loads as compared to 1.6 to 2.5 mm puncture diameters. 3 and 3.5 mm puncture diameters recovered 50 and 12% of their initial height, respectively. In contrast 1.6-2.5 mm puncture diameters recovered about 60-80% of their original height (Fig. 7).

This indicates the advantage of using a small diameter needle such as a needle having an outer diameter of 1.6, 2 and 2.5 mm (16, 14 and 13G) for the direct application of a substance into a target tissue or organ, since the damage inflicted on the surrounding tissues is minimized. It is of crucial importance to use a small diameter needle when the substance is applied to organs which are subjected to high pressure.

### Example 4: Injection of Fibrin Sealant into Isolated Intervertebral Discs using a Needle having an Outer Diameter of 0.8192 mm (21G Needle)

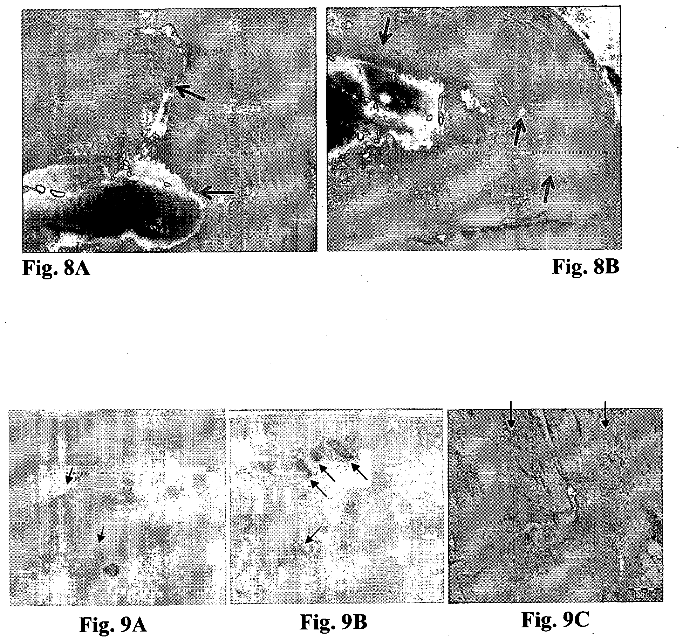

As indicated above fibrin sealant can be used to restore or increase the height of the disc. The objective of the experiment was to determine whether the fibrin sealant can penetrate into the NP region following injection with a needle having an outer diameter of 0.8192 mm (21G needle). For this purpose, pig isolated lumbar spine (L1-L5) was cleaned from muscle tissue and lumbar vertebral bodies were cut in the middle using an electric bone saw to obtain isolated IVDs. The isolated IVDs were emptied from their nucleus pulposus tissue by injection of 0.5 ml digestion solution containing 2 mg/ml hyaluronidase in PBS. After 30 minutes, the IVDs were washed with PBS and EDTA as specified in Example 3. Afterwards, the emptied-isolated IVDs were inflicted with annulus fibrosus defects as follows: a 1.6 mm puncture diameter hole was done in the annulus fibrosus tissue using a needle having an outer diameter of 1.651 mm (a 16G needle). Then, a scalpel was inserted through the created hole to obtain full annular tears and annular fissures simulating grade 1 and 2 degenerative discs [characterized according to the "modified Dallas Discogram" naming system (Sachs et al. "Dallas discogram description. A new classification of CT/discography in low-back disorders". Spine. 1987;12:287-294]. Following this step, the scalpel was removed and a 2 mm metal screw was inserted into the hole. Next, fibrin sealant (like the one described above) containing indigo carmine dye (0.15 mg/ml in 100 IU thrombin; Sigma cat No 13116-4) was injected into the nuclear space of the emptied-isolated intervertebral discs using a needle having an outer diameter of 0.8192 mm (a 21G needle). The sealant was injected in a different location until excess solution was spilt out of the injection site. Fig. 8 shows the localization of the fibrin sealant following the injection procedure in discs having full annular tears (A) and annular fissures simulating grade 1 and 2 degenerative discs (B). The localization of the sealant is marked with an arrow. The results clearly show that fibrin sealant can penetrate into the NP region following the injection procedure and can fill annular fissures and tears.

**Example 5: Injection of Fibrin Sealant into the Intravertebral Discs in an *in-vivo* setting using the Concentric Needle Arrangement**

The following experiment was carried out to explore the ability of the concentric needle arrangement to inject fibrin sealant into the intravertebral disc space in an *in vivo* setting. The ease of use and the function of the concentric needle arrangement during the injection procedure were also evaluated.

For this purpose, fibrin sealant was injected into the intervertebral disc of a pig using the concentric needle arrangement of the invention. An *in vivo* injection of PBS with a standard 21G spinal needle having an outer diameter of 0.8192 was used as the control. The injection device used to inject the fibrin sealant and the PBS is described below.

Injection Device used to inject the fibrin sealant. Omrix's applicator device (as described in WO-A-2007/059801 excluding the Y mixing module) was connected to a connecting element 20 and to a concentric lumen arrangement 12 as shown in Fig. 1. The connection was carried out with two flexible connective tubes with luer lock at ends. The applicator device was incased in an outer shell of a pressing mechanism which contained a screw that enabled to apply a gradual and a continuous force to the plungers of the injection device (38, 40 in Fig. 1). To monitor the pressure applied during the injection procedure, the device included a load cell pressure transducer (Burster 9201-v001). For visual display, the transducer was connected to a computer system via flexible cords.

The concentric needle arrangement was custom made by Tegra Medical from a thin wall outer needle having an outer diameter of 1.651 mm and an inner diameter of 1.3589 mm (a 16G needle), and an inner needle having an outer diameter of 0.8192 mm and inner diameter of 0.514 mm (a 21G needle). The outer needle was connected and laser welded at its end to the inner needle. The inner needle further extends the outer needle by 20 mm and the opening (0.2 x 1.5 mm) present in the inner needle is located 25 mm before the end of the tip of the inner needle. That means that the two components flow together in a distance of 25 mm before they are expelled from the projecting inner needle.

PBS was injected with the same injection device excluding the connecting element 20 and the concentric lumen arrangement 12. Instead a standard 21G spinal needle was used ("phoenix" spinal needles sterilized by ethylene oxide Kobayashi Shoji K.K Tokyo, Japan). The outlets of the two cylinders were connected to a one extension connective tube using a 3-WAY STOPCOCK.

Fibrin Sealant. Evicel™, fibrin glue manufactured by OMRIX Biopharmaceuticals LTD., was used in these experiments. The thrombin component was diluted 10-fold (to about 100 IU/ml) directly before use with thrombin dilution buffer (NaCl 150 mM, sodium acetate 20 mM, mannitol 2%, human serum albumin 6 mg/ml, and $CaCl_2$ 40mM). The BAC component was used as is.

Before the injection procedure, the two components were thawed at 37°C for 15 minutes, and placed on a roller for 30 minutes at room temprature.

A contrasting agent (Iopamiro 370; Bracco s.p.a) was added into the thrombin component at a concentration of 30% before the injection procedure (a final concentration of 15% in the mixed fibrin glue).

Saline. 15% Iopamiro was added to the saline before the injection.

Experimental animals. Two female domestic crossbred pig (Sus scrofa) weighing 51 and 53 kg were used. The animals were handled and maintained in accordance with the current ethical requirements in an authorized facility. The animals underwent a physical examination preoperatively. The following parameters were evaluated body weight, temperature, heart rate and respiratory rate.

During the procedure the animals were continually monitored with controlled respiratory support and fluid support.

Anesthetic Regimen. Anesthesia was induced with an intramuscular mixture of ketamine (15-25 mg/kg), xylazine (2 mg/kg) and Diazepam (0.5-10 mg/kg). This mixture was administered until a sufficient level of anesthesia was obtained to allow endotracheal intubation. The endotracheal tube was then attached to a veterinary anesthesia machine. Anesthesia for the remainder of the preparation and surgical procedure was maintained by semi-closed circuit inhalation of 1-3% isoflurane with an oxygen flow of 11 - 15 ml/kg/minuts. Fluid Therapy. Intravenous access was established and Lactated Ringer's solution was administered throughout the procedure at a rate of approximately 10 - 11 ml/kg/hr. ECG, pulse, and blood pressure were monitored during the procedure.

Surgical Procedure. Depilation of the dorsal area was accomplished with an electric animal clipper equipped with a surgical shaving blade. The area was vacuumed to remove hair clippings and debris. The entire animal body was covered with a drape. The surgical procedure was preformed under anesthesia as described above. The animal was laid on its anterior side and fibrin sealant or saline containing contrasting agent were injected into the IVD apace as elaborated below. A fluorography (General electric OEC9900) was used as a visual guidance to locate the IVD cavity and to view the location and distribution of the injected material.

Injection procedure.

Each pig was injected with both saline and fibrin sealant as follows:

1. A 21G spinal needle was positioned close to the pig's IVD (L4-L5) and then attached to the connective tube of the injection device as described above. Then, the needle was inserted into the nucleus pulposus region of the disc and saline containing a contrast agent was injected therein. The injection was stopped when the pressure monitor indicated 344,737 Pa (50 PSI).

2. A concentric needle arrangement connected to connecting element 20 was positioned close to the IVD (L2-L3). In the next step, the needle arrangement was attached to two connective tubes of an injection device as described above. The two connective tubes were filled with the fibrin glue components up to their tips prior to their attachment with the connecting element. A very small amount (present at the tip of the needle) of the two components was injected until the contrast agent was seen in the fluorography exiting the tip of the inner needle. The concentric needle was then inserted through the annulus fibrosis and into the nucleus pulposus region of the disc, and fibrin sealant containing a contrast agent was injected until the pressure reached 344,737 Pa (50 PSI).

The second pig was injected with saline and fibrin sealant in the same manner only this time L2-L3 IVD were injected with saline and L4-L5 IVD were injected with fibrin sealant. In both pigs L1-L2 received no treatment.

Following the surgical procedure and up to euthanasia the animals were monitored to assess any signs of pain or abnormal behavior. They were examined weekly for body weight, behavior and local signs at the site of injection. Special attention was given to the ability of the animal to walk following the surgery and cases of limping were monitored.

The animals were euthanized 28 days after the injection procedure by an IV injection of pentobarbital sodium (0.3 ml/kg). The spine back (from thorax to sacrum) was removed with surrounding tissue (muscle tissue), pelvic and femoral bones. The spine and surrounding tissue were placed in a container with chillers until analysis.

Disc isolation procedure. After about 4 hours the IVDs (L1-L2, L2-L3, L4-L5) of both spines were cut in the middle using an electric saw to obtain isolated IVDs, and placed in a sealed plastic tube containing 120 ml fresh formalin for 4 days at room temperature. The lumbar vertebrae were numbered according to their location compared with the sacrum.

Fixation and section preparation. The isolated IVDs were placed in 10% formalin solution for at least 4 days. In the next step, the IVDs were removed from the formalin solution into a decalcifying solution (Calci-clear; national diagnostics rapid; cat HS 105) for 3 to 4 days. The IVDs were then dehydrated in four steps by gentle agitation in increasing ethanol concentrations from 70% to 100%. Subsequently, the IVDs were washed in Histoclear (gadot cat. No. 5989-27-5) at room temperature (20-25°C) for 30 min and then embedded in paraffin at 56-60°C for 1 hour. 6 $\mu$M sections were cut from the NP region using a microtome, the sections were rinsed in 70% ethanol, placed in a water bath at 40° C for up to 10 min and loaded on a glass slide. The slides were left to dry at 37° C for 1 hour.

Immunostaining. The presence of fibrin within the intervertebral disc space was determined by an immunostaining. The slides were incubated in PBS for 5 minutes at room temperature with gentle agitation. This procedure was repeated four times. Then, the slides were incubated in IF buffer [5ml 5% fetal calf serum (FCS) and 1 g 2% BSA in PBS] for 30 minutes at room temperature and washed with PBS for 5 minutes. In the next step, the slides were incubated with goat anti-fibrinogen (Sigma cat. No. F8512; Diluted 1:100 in IF buffer) for about 75 minutes and washed four times with PBS for 5 minutes. The slides were incubated for 30 minutes with a secondary anti-goat antibody (Sigma cat. No. A7650; 1:2000 in IF buffer) and washed four times for 5 minutes with PBS. To visualize the antibody binding, the sections were developed using "Sigma Fast Red" kit (cat. No. N3020). The kit was used according to the manufacturer instructions. Stained casted fibrin clots preformed *in vitro* served as reference. The casted fibrin clots were preformed by mixing an equal volume of the diluted thrombin component (1:10 with thrombin dilution buffer) and the BAC component (a total of 0.5 ml). The clots were left to polymerize for about 30 min.

Hematoxylin and Eosin (H&E) staining. Damage to the injected nucleus pulposus region was determined by H&E staining which stains the nucleus and the cytoplasm. The slides were submerged in haematoxylin solution (Sigma cat. No. MHS32) for 10 minutes and washed with tap water for 10 minutes. The slides were then placed in Eosin solution (Sigma cat. No. E4382) for 30 seconds and washed with tap water. In the next step, the slides were dehydrated by immersion in increasing ethanol concentrations (70% to 100%). Subsequently, the slides were washed with Histoclear and analyzed microscopically.

Alcian blue staining. The slides were covered with alcian blue solution (1g alcian blue dissolved in 100 ml 3% acetic acid; Fluka cat. No. 45731) for 3 minutes. The alcian blue solution was then removed and remnants were gently absorbed using a paper towel. Subsequently, the slides were washed for distaining with 5% acetic acid for 10 minutes followed by a wash with demineralized water for 5 min.

The surgeon reported that the intradiscal injection using an injection device comprising the concentric lumen needle arrangement according to the invention was carried out without any complication. In addition, the surgeon reported that the concentric needle had increased resistance to bending when compared to the needle used for the PBS injection (a needle having an outer diameter of 0.8192).

One day following the procedure both pigs were able to stand-up and move freely in their cages. No deleterious effects of the procedure were observed and no pain medications were required. No pathological findings were observed in the nucleus pulposus region of the pigs injected with the concentric needle arrangement or with the 21G spinal needle (having an outer diameter of 0.8192) as determined by H&E staining (data not shown).

The animals gained weight throughout the experimental period. On day 28 the animal's weight was 65 and 61.5 (they gained 10.5-12 kg).

Fig. 9A shows immunostaining of fibrin in a representative section obtained from an IVD injected with the fibrin sealant components. The immunostained casted fibrin clots used to verify the fibrin staining are shown in Fig. 9B. Positive fibrin staining is marked with arrows.

It is apparent that fibrin was present in the stained sections indicating that using an applicator device comprising a concentric needle arrangement according to the invention can be effectively used to penetrate into the NP region in an *in vivo* setting and to inject fibrin within the intervertebral disc space.

These results were confirmed in another set of experiment wherein 15 animals were injected with fibrin into their intravertebral disc space with an injection device comprising a concentric lumen arrangement as described above. The experiment was carried out in the same manner specified above with the following modifications: Omnipaque™ (GE Healthcare; Cat. No. NDC 0407-1413-51) at a final concentration of 30% was used as the contrast agent, the solutions (fibrin components or saline) were injected until a pressure of 413,685 Pa (60 PSI) was reached, and the animals were euthanized 3 days after the injection procedure.

The disc was extracted, isolated IVDs were obtained, and 6 $\mu$M sections were cut from the NP region and loaded on a glass slide according to the procedures specified above. In the next step, the presence of fibrin within the intervertebral disc was checked by immunostaining (see procedure above). Prior to the immunostaining the slides were counterstained with alcian blue solution as described above.

Fig. 9C shows immunostaining of fibrin in a representative section obtained from an IVD injected with the fibrin sealant components in the second study preformed. Fibrin staining is marked with arrows.

The obtained results are in line with the previous results. It was found that fibrin was present within the intervertebral disc space and thus the concentric needle arrangement is suitable for injection of a substance into the intervertebral disc.

## Example 6: Evaluating the Damage Inflicted to the Annulus Fibrosus tissue by Different Needle Gauges

Example 3 showed that the puncture diameter created during the injection procedure affects the height restoration ability following compressive loads.

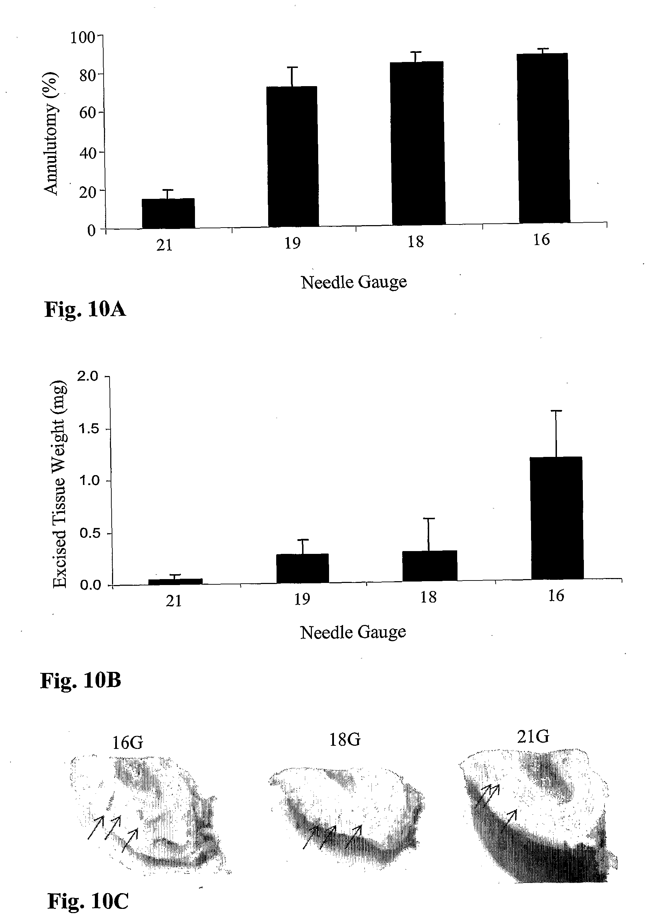

In order to further evaluate the influence of the injection on the surrounding tissue, the effect of the needle gauge on the extent of annulus fibrosus tissue injury (annulutomy) was assessed. For this purpose, isolated pig lumbar spine was cleaned from muscle tissue and the annulus fibrosus tissue was punctured with different gauge needles (about 5 stabs for each needle to prevent blunting of the needle). Subsequent to each stabbing, a metal rod was inserted through the needle and the annulus material accumulated in the needle was collected and weighed. The weight of the total excised annulus tissue was divided by the number of stabs so that the tissue excised per stabbing could be calculated. The percentage of punctures which resulted in annulus fibrosus tissue removing (annulotomy) and the excised tissue weight per stabbing for each gauge size are presented in Figs. 10A and B, respectively. The results represent the means $\pm$ S.D. of three to four independent experiments.

The results show that tissue removing subsequent stabbing is proportional to the needle diameter, i.e. lower diameter needle results in less tissue removing.

When observing the IVD sections following the puncturing procedure (Fig. 10C), it is evident that puncturing with a higher gauge needle (lower diameter needle) inflicts less damage to the annulus fibrosus structure as compared with a lower gauge needle (higher diameter needle). The ruptures in the annulus fibrosus tissue are marked with an arrow.

These results indicate that using a large-needle gauge will decrease the extent of tissue damage subsequent the injection procedure.

## Example 7: The Resistance of the Concentric Needle to Fluid Flow

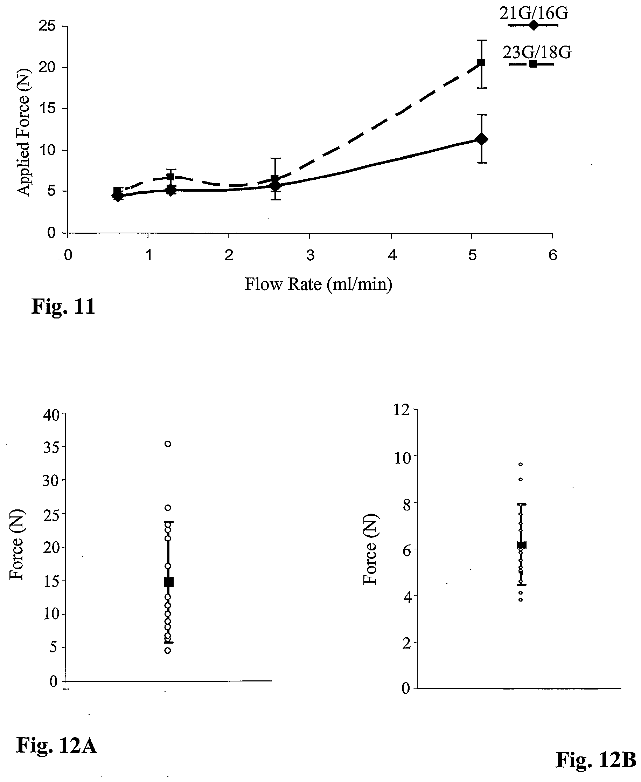

The present example was carried out to investigate the flow characteristics of the concentric needle. For this purpose, Omrix's injection device (as described in WO-A-2007/059801 excluding the Y mixing module) was filled with Fibrin Sealant (like the one described above). 2 ml of each component was applied in a separate syringe. Afterwards, the concentric needles [(outer diameter 0.8192 mm/1.651mm (21G/16G) or 0.6414mm/1.270mm (23G/18G) (inner/outer)] were connected to the device and the resistance of the concentric needle to flow was measured. The requested flow rate was fed (0.5 to 5 ml/min) to the tension and compression testing machine and the maximal force which was needed in order to achieve each flow rate was recorded for each concentric needle type (Fig. 11). The force applied to the device resulted in movement of the plungers and consequently expelling of the sealant from the syringe. The results represent the means $\pm$ S.D. of three independent experiments.

It is evident that the concentric needle having an outer diameter of 0.6414mm/1.270mm (23G/18G) has a higher resistance to flow as compared to the 0.8192 mm/1.651mm (21G/16G) needle.

In addition, it is apparent that a higher force was required in the 0.6414mm/1.270mm (23G/18G) concentric needle as compared to the 0.8192 mm/1.651mm (21G/16G) concentric needle in order to achieve the same flow rate. For example, if a flow of 5 ml/min is desired, a force of 20 and 11 N is required in the 0.6414mm/1.270mm (23G/18G) and 0.8192 mm/1.651mm (21G/16G) needles, respectively.

## Example 8: The Force Required for Unclogging the Needle

When the two components are mixed together polymerization process is activated and clogging of the device may occur. Thus, the following example was carried out in order to investigate the force required to unclog the concentric needle.

Omrix's injection device as described in WO-A-2007/059801 excluding the Y mixing module was filled with Fibrin Sealant (like the one described above). 2 ml of each component was applied in a separate syringe. Afterwards, 0.8192 mm/1.651mm (21G/16G) or 0.6414mm/1.270mm (23G/18G) concentric needles were connected to the device. The sealant was expelled from the syringes until excess solution was spilt from the needle, indicating that the needle was filled.

The injection was stopped for 2 min and force was applied to the plungers until flow was resumed. The force was measured using the LF plus (LLOYD) instrument. Fig. 12 shows the maximal force needed to overcome needle clogging in 0.6414mm/1.270mm (23G/18G; A) and 0.8192 mm/1.651mm (21G/16G; B) concentric needles. The results represent means $\pm$ S.D. of 15 measurements for each needle.

The results demonstrate that the force required to unclog the 0.6414mm/1.270mm (23G/18G) needle was significantly higher than in the 0.8192 mm/1.651mm (21G/16G) needle (15 vs. 6N, respectively). The upper limit of the dispersion measurements was about 35 and 9.5 N for the 0.6414mm/1.270mm (23G/18G) and 0.8192 mm/1.651mm (21G/16G) concentric needles, respectively.

These results indicate that a partially set up clot within the inner needle can be successfully expelled out of the needle when a reasonable force is applied. Although the invention has been described and illustrated with reference to specific illustrative embodiments thereof, it is not intended that the invention be limited to those illustrative embodiments. Those skilled in the art will recognize that variations and modifications can be made without departing from the scope of the claims that follow. It is therefore intended to include within the invention all such variations and modifications as fall within the scope of the appended claims.

## Claims

**1.** A device for administering a surgical sealant comprising at least two components wherein a first component comprises fibrinogen and a second component comprises thrombin, wherein the device comprises:

- a concentric lumen arrangement (12) including (i) an inner cannula (14) having a lumen (16) with an inlet opening at a first end and a tip with an outlet opening at a second end (62) opposite its first end, and (ii) an outer sheath (18) having opposite first and second ends facing the respective first and second ends of the inner cannula, and surrounding the inner cannula along an axial length between the first and second ends of the inner cannula,
- wherein the outer sheath at its second end (60) is sealingly and fixedly connected to the inner cannula and defines an outer lumen (28) around the inner cannula,
- wherein the second end (60) of the outer sheath is truncated and spaced apart from the tip end of the inner cannula (14), and
- wherein the inner cannula is provided with one hole (66) located 3 mm to 50 mm from the tip of the inner cannula for providing fluid communication between the outer lumen (28) around the inner cannula and the lumen of the inner cannula (16)
- wherein the device is configured such that the first and second component flow separately through the inner and outer lumen and are mixed in the inner cannula prior to being administered.

**2.** The device according to claim 1, further comprising at least a first (32) and a second (30) storage containers for storing the components of the substance, wherein the containers are capable of being connected to the concentric lumen arrangement (12) with the first container in fluid communication with the lumen of the inner cannula (16) and the second container in fluid communication with the outer lumen (28) around the inner cannula (14).

3. The device according to claim 2, further comprising a connecting element (20) for providing fluid communication between the containers (30, 32) and the concentric lumen arrangement (12).

4. The device according to claim 3, wherein the connecting element (20) comprises a first port (24) for connecting to the first container (32) and a second port (26) for connecting to the second container (30), wherein the first port is in fluid communication with the inlet opening of the inner cannula and the second port is in fluid communication with the outer lumen at the first end of the outer sheath.

5. The device according to claim 4, wherein the connecting element (20) further comprises a holding element (22) for holding the inner cannula and outer sheath of the concentric lumen arrangement, wherein the holding element comprises an inner hollow space (58) through which the concentric lumen arrangement extends, with the first end of the outer sheath located within the hollow space and the inner cannula further extending through the terminal element, and wherein a connecting channel (56) is provided starting from the second port (26) and terminating in the inner hollow space of the terminal element.

6. The device according to any one of claims 1 to 5, wherein the hole of the inner cannula (66) is arranged adjacent the second end of the outer sheath (60).

7. The device according to any one of claims 2 to 5, wherein the concentric lumen arrangement further comprises at least one further sheath (68) surrounding the outer sheath (18) along an axial length thereof and comprising first and second ends facing the first and second ends of the outer sheath, respectively, wherein the further sheath is sealingly connected (61) to the outer sheath (18) adjacent the second end thereof and defines a further lumen (70) around the outer sheath, and wherein the outer sheath is provided with at least a second opening (72) providing fluid communication between the further outer lumen (70) around the outer sheath (18) and the lumen (28) within the outer sheath around the inner cannula, and wherein at least one further storage container is provided for storing a further component of the substance and capable of being connected to the first end of the at least one further sheath of the concentric lumen arrangement.

8. The device according to any one of claims 1 to 7, further comprising a pressing mechanism (46) for pressing the components out of the containers (30, 32) to the concentric lumen arrangement (12) and further therethrough out of the tip at the second end of the inner cannula (62).

9. The device according to any one of claims 1 to 8, wherein the administering is carried out by injection.

10. The device according to any one of claims 1 to 8, wherein the administering is carried out by spraying or dripping.

**Patentansprüche**

1. Vorrichtung zur Verabreichung eines chirurgischen Dichtungsmittels, das mindestens zwei Komponenten umfasst, wobei eine erste Komponente Fibrinogen umfasst und eine zweite Komponente Thrombin umfasst, wobei die Vorrichtung umfasst:

   - eine konzentrische Lumenanordnung (12), aufweisend (i) eine Innenkanüle (14), die ein Lumen (16) mit einer Einlassöffnung an einem ersten Ende und eine Spitze mit einer Auslassöffnung an einem zweiten Ende (62) gegenüber ihrem ersten Ende aufweist, und (ii) eine Außenhülle (18), die gegenüberliegende erste und zweite Enden, die den jeweiligen ersten und zweiten Enden der Innenkanüle zugewandt sind, aufweist und die Innenkanüle entlang einer axialen Länge zwischen den ersten und zweiten Enden der Innenkanüle umgibt,
   - wobei die Außenhülle an ihrem zweiten Ende (60) abdichtend und fest mit der Innenkanüle verbunden ist und ein Außenlumen (28) um die Innenkanüle definiert,
   - wobei das zweite Ende (60) der Außenhülle verkürzt ist und von dem Spitzenende der Innenkanüle (14) beabstandet ist, und
   - wobei die Innenkanüle zur Bereitstellung einer Fluidverbindung zwischen dem Außenlumen (28) um die Innenkanüle und dem Lumen der Innenkanüle (16) ein Loch (66) aufweist, das 3 mm bis 50 mm von der Spitze der Innenkanüle angeordnet ist,
   - wobei die Vorrichtung so ausgelegt ist, dass die ersten und zweiten Komponenten getrennt durch das Innen- und Außenlumen fließen und vor der Verabreichung in der Innenkanüle gemischt werden.

2. Vorrichtung nach Anspruch 1, ferner umfassend mindestens einen ersten (32) und einen zweiten (30) Aufbewahrungsbehälter zur Aufbewahrung der Komponenten der Substanz, wobei die Behälter mit der konzentrischen Lumenanordnung (12) verbunden werden können, so dass der erste Behälter mit dem Lumen der Innenkanüle (16) in Fluidverbindung steht und der zweite Behälter mit dem Außenlumen (28) um die Innenkanüle (14) in Fluidverbindung steht.

3. Vorrichtung nach Anspruch 2, ferner umfassend ein Verbindungselement (20) zur Bereitstellung einer Fluidverbindung zwischen den Behältern (30, 32) und der konzentrischen Lumenanordnung (12).

4. Vorrichtung nach Anspruch 3, wobei das Verbindungselement (20) eine erste Öffnung (24) zur Verbindung mit dem ersten Behälter (32) und eine zweite Öffnung (26) zur Verbindung mit dem zweiten Behälter (30) umfasst, wobei die erste Öffnung mit der Einlassöffnung der Innenkanüle in Fluidverbindung steht und die zweite Öffnung mit dem Außenlumen am ersten Ende der Außenhülle in Fluidverbindung steht.

5. Vorrichtung nach Anspruch 4, wobei das Verbindungselement (20) ferner ein Halteelement (22) zum Halten der Innenkanüle und der Außenhülle der konzentrischen Lumenanordnung umfasst, wobei das Halteelement einen inneren Hohlraum (58) umfasst, durch den sich die konzentrische Lumenanordnung erstreckt, wobei das erste Ende der Außenhülle innerhalb des Hohlraums angeordnet ist und sich die Innenkanüle ferner durch das terminale Element erstreckt, und wobei ein Verbindungskanal (56) bereitgestellt ist, der an der zweiten Öffnung (26) beginnt und im inneren Hohlraum des terminalen Elements endet.

6. Vorrichtung nach einem der Ansprüche 1 bis 5, wobei das Loch der Innenkanüle (66) neben dem zweiten Ende der Außenhülle (60) angeordnet ist.

7. Vorrichtung nach einem der Ansprüche 2 bis 5, wobei die konzentrische Lumenanordnung ferner mindestens eine weitere Hülle (68) umfasst, die die Außenhülle (18) entlang einer axialen Länge davon umgibt und erste und zweite Enden umfasst, die den ersten bzw. zweiten Enden der Außenhülle zugewandt sind, wobei die weitere Hülle abdichtend mit der Außenhülle (18) neben dem zweiten Ende davon verbunden (61) ist und ein weiteres Lumen (70) um die Außenhülle definiert, und wobei die Außenhülle mindestens eine zweite Öffnung (72) aufweist, die eine Fluidverbindung zwischen dem weiteren Außenlumen (70) um die Außenhülle (18) und dem Lumen (28) innerhalb der Außenhülle um die Innenkanüle bereitstellt, und wobei mindestens ein weiterer Aufbewahrungsbehälter zur Aufbewahrung einer weiteren Komponente der Substanz bereitgestellt ist, und der mit dem ersten Ende der mindestens einen weiteren Hülle der konzentrischen Lumenanordnung verbunden werden kann.

8. Vorrichtung nach einem der Ansprüche 1 bis 7, ferner umfassend einen Druckmechanismus (46) zum Herausdrücken der Komponenten aus den Behältern (30, 32) zur konzentrischen Lumenanordnung (12) und ferner dort hindurch aus der Spitze am zweiten Ende der Innenkanüle (62).

9. Vorrichtung nach einem der Ansprüche 1 bis 8, wobei die Verabreichung durch Injektion durchgeführt wird.

10. Vorrichtung nach einem der Ansprüche 1 bis 8, wobei die Verabreichung durch Sprühen oder Auftropfen durchgeführt wird.

**Revendications**

1. Dispositif pour administrer un produit d'étanchéité chirurgical comprenant au moins deux composants, un premier composant comprenant du fibrinogène et un second composant comprenant de la thrombine, le dispositif comprenant :

- un agencement de lumière concentrique (12) comprenant (i) une canule intérieure (14) ayant une lumière (16) avec une ouverture d'entrée à une première extrémité et un embout avec une ouverture de sortie à une seconde extrémité (62) opposée à sa première extrémité, et (ii) une gaine extérieure (18) ayant des première et seconde extrémités opposées faisant face aux première et seconde extrémités respectives de la canule intérieure, et entourant la canule intérieure le long d'une longueur axiale entre les première et seconde extrémités de la canule intérieure,
- la gaine extérieure à sa deuxième extrémité (60) étant raccordée de manière étanche et fixe à la canule intérieure et définissant une lumière extérieure (28) autour de la canule intérieure,

- la seconde extrémité (60) de la gaine extérieure étant tronquée et espacée de l'extrémité d'embout de la canule intérieure (14), et
- la canule intérieure étant pourvue d'un trou (66) situé de 3 mm à 50 mm de l'embout de la canule intérieure pour assurer une communication fluidique entre la lumière extérieure (28) autour de la canule intérieure et la lumière de la canule intérieure (16),
- le dispositif étant configuré de telle sorte que le premier et le second composant s'écoulent séparément à travers la lumière intérieure et extérieure et sont mélangés dans la canule intérieure avant d'être administrés.

2. Dispositif selon la revendication 1, comprenant en outre au moins un premier (32) et un second (30) récipients de stockage pour stocker les composants de la substance, les récipients étant capables d'être raccordés à l'agencement de lumière concentrique (12) avec le premier récipient en communication fluide avec la lumière de la canule intérieure (16) et le second récipient en communication fluide avec la lumière extérieure (28) autour de la canule intérieure (14).

3. Dispositif selon la revendication 2, comprenant en outre un élément de raccordement (20) pour assurer une communication fluidique entre les récipients (30, 32) et l'agencement de lumières concentriques (12).

4. Dispositif selon la revendication 3, l'élément de raccordement (20) comprenant un premier orifice (24) pour le raccordement au premier récipient (32) et un second orifice (26) pour le raccordement au second récipient (30), le premier orifice étant en communication fluidique avec l'ouverture d'entrée de la canule intérieure et le second orifice étant en communication fluidique avec la lumière extérieure à la première extrémité de la gaine extérieure.

5. Dispositif selon la revendication 4, l'élément de raccordement (20) comprenant en outre un élément de maintien (22) pour maintenir la canule intérieure et la gaine extérieure de l'agencement de lumière concentrique, l'élément de maintien comprenant un espace creux intérieur (58) à travers lequel l'agencement de lumière concentrique s'étend, avec la première extrémité de la gaine extérieure située à l'intérieur de l'espace creux et la canule intérieure s'étendant en outre à travers l'élément terminal, et un canal de raccordement (56) étant fourni à partir du second port (26) et se terminant dans l'espace creux intérieur de l'élément terminal.

6. Dispositif selon l'une quelconque des revendications 1 à 5, le trou de la canule intérieure (66) étant agencé de manière adjacente à la seconde extrémité de la gaine extérieure (60).

7. Dispositif selon l'une quelconque des revendications 2 à 5, l'agencement de lumière concentrique comprenant en outre au moins une autre gaine (68) entourant la gaine extérieure (18) le long d'une longueur axiale de celle-ci et comprenant des première et seconde extrémités faisant face respectivement aux première et seconde extrémités de la gaine extérieure, l'autre gaine étant raccordée de manière étanche (61) à la gaine extérieure (18) adjacente à la seconde extrémité de celle-ci et définissant une autre lumière (70) autour de la gaine extérieure, et la gaine extérieure étant pourvue d'au moins une seconde ouverture (72) fournissant une communication fluidique entre la lumière extérieure supplémentaire (70) autour de la gaine extérieure (18) et la lumière (28) à l'intérieur de la gaine extérieure autour de la canule intérieure, et au moins un récipient de stockage supplémentaire étant prévu pour stocker un composant supplémentaire de la substance et capable d'être raccordé à la première extrémité de l'au moins une gaine supplémentaire de l'agencement de lumière concentrique.

8. Dispositif selon l'une quelconque des revendications 1 à 7, comprenant en outre un mécanisme de pression (46) pour presser les composants hors des récipients (30, 32) vers l'agencement de lumière concentrique (12) et en outre à travers celui-ci hors de l'embout à la seconde extrémité de la canule intérieure (62).

9. Dispositif selon l'une quelconque des revendications 1 à 8, l'administration étant effectuée par injection.

10. Dispositif selon l'une quelconque des revendications 1 à 8, l'administration étant effectuée par pulvérisation ou goutte à goutte.

**Fig. 1**

**Fig. 2**

**Fig. 3**

**Fig. 4**

**Fig. 5**

**Fig. 6**

Puncture Diameter (mm)

**Fig. 7**

**Fig. 8A**

**Fig. 8B**

**Fig. 9A**

**Fig. 9B**

**Fig. 9C**

**Fig. 10A**

**Fig. 10B**

**Fig. 10C**

Fig. 11

Fig. 12A

Fig. 12B

**Fig. 13**

**Fig. 14**

**Fig. 15A**          **Fig. 15B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6565539 B
- US 6464663 B
- US 6234994 B
- US 6113571 A
- WO 2007059801 A
- EP 1091694 B
- EP 0654976 B
- US 20080103564 A
- US 20080060970 A
- US 20070213660 A
- WO 2008103296 A
- WO 9728834 A
- US 5814022 A
- EP 0139091 A
- US 5833652 A
- US 6254587 B
- US 4897079 A
- US 4959058 A
- US 5984889 A
- EP 0537573 B
- WO 2008092029 A
- WO 2008106357 A
- WO 8404043 A
- EP 0534178 B
- WO 9305822 A
- EP 0378798 B
- US 6121232 B
- WO 9833533 A
- EP 1390485 B
- US 7641918 B
- EP 0814866 B
- WO 2006086510 A

**Non-patent literature cited in the description**

- **DERBY ; KIM BJ.** Effect of intradiscal electrothermal treatment with a short heating catheter and fibrin on discogenic low back pain. *Am J Phys Med Rehabil,* 2005, vol. 84, 560-561
- **SACHS et al.** Dallas discogram description. A new classification of CT/discography in low-back disorders. *Spine,* 1987, vol. 12, 287-294